# EUROPÄISCHE PATENTANMELDUNG

(11) **EP 3 461 534 A1**
(43) Veröffentlichungstag der Anmeldung: **03.04.2019**
(21) Anmeldenummer: 18185529.7
(22) Anmeldetag: 25.07.2018
(51) Int. Cl.: A61N 1/375, A61N 1/37, A61N 1/08

(54) **AUSSENHÜLLENTEIL EINES IMPLANTIERBAREN MEDIZINELEKTRONISCHEN GERÄTES**

(30) Priorität: 29.09.2017 EP 17193996
(71) Anmelder: BIOTRONIK SE & Co. KG, 12359 Berlin (DE)
(72) Erfinder: Kronmüller, Daniel, 90409 Nürnberg (DE); Sontheimer, Thomas, 90574 Roßtal (DE)
(74) Vertreter: Keck, Hans-Georg

(57) **Zusammenfassung**

Metallisches Außenhüllenteil eines implantierbaren medizinelektronischen Gerätes, das mindestens einen inneren Hohlraum und/oder nichtleitende Einschluss oder Abschnitt mit einer Vielzahl kleiner Hohlräume und/oder nichtleitende Einschlüsse aufweist, der mindestens zur Geräteaußenseite hin mit einer geschlossenen Metallschicht hermetisch dicht abgeschlossen ist.

## Beschreibung

Die Erfindung betrifft ein metallisches Außenhüllenteil eines implantierbaren medizinelektronischen Gerätes. Insbesondere betrifft sie einen Durchführungs-Flansch eines solchen Gerätes oder die Gehäusehalbschalen, welche im gefügten Zustand die Außenhülle des medizinelektronischen Gerätes bilden. Weiterhin betrifft sie ein medizinelektronisches Gerät selbst, insbesondere Herzschrittmacher oder Kardioverter oder ein Cochlearimplantat oder einen Neurostimulator.

Zur Therapie von Rhythmusstörungen des Herzens sind implantierbare medizinelektronische Geräte, wie etwa Herzschrittmacher oder implantierbare Defibrillatoren, seit langem bekannt und im massenhaften Einsatz. In den letzten Jahren haben auch neuartige implantierbare Geräte, wie etwa Neurostimulatoren oder Cochlearimplantate, breite Anwendung gefunden. Solche Geräte haben typischerweise eine metallische Außenhülle, die hermetisch dicht sein und elektrische und elektronische Funktionseinheiten im Inneren des Gerätes dauerhaft vor Körperflüssigkeiten schützen muss. Zugleich bietet eine solche Außenhülle einen Schutz vor elektromagnetischer Störstrahlung (EMV, engl. EMI) aus der Lebensumgebung des Patienten oder bei bildgebenden Diagnoseverfahren.

Die meisten praktisch bedeutsamen implantierbaren medizinelektronischen Geräte sind dazu vorgesehen, über geeignete Sensoren Signale des Körpers zu messen oder Nerven bzw. Gewebe aktiv zu stimulieren. Um diese Funktion auszuführen, sind im Gehäuse des Gerätes elektronische/elektrische Funktionseinheiten zur Verarbeitung der Daten und/oder zur geeigneten Steuerung der Reizerzeugung untergebracht. Häufig werden die Sensoren außerhalb des medizinelektronischen Gerätes über mindestens eine Elektrodenleitung angeschlossen.

Damit der Patient durch das Tragen des Implantates nicht beeinträchtigt wird, sind die elektronischen/elektrischen Funktionseinheiten durch eine biokompatible hermetisch dichte Außenhülle gekapselt. Diese verhindert zum einen, dass der Organismus Werkstoffe der Elektronik angreift oder resorbiert und so allmählich durch Schwermetalle, wie zum Beispiel Blei oder Kupfer vergiftet wird. Auf der anderen Seite wird durch die hermetische Kapselung verhindert, dass die Elektronik durch Flüssigkeiten des Organismus geschädigt wird und so die funktionalen Fähigkeiten verliert. Die Außenhülle ist aus mehreren Teilen gefügt. Hierbei handelt es sich häufig um Gehäusehalbschalen, welche nach Bedarf um elektrische Durchführungen und/oder einen sogenannten Header ergänzt werden.

Die Überprüfung der Hermetizität erfolgt anhand der Leckrate, welche beispielsweise durch Lecktests bestimmt werden kann. Entsprechende Lecktests können beispielsweise mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, können diese maximal zulässigen Helium-Leckraten beispielsweise von 1 × 10⁻⁸ mbar·L/sec bis 1 × 10⁻⁷ mbar·L/sec betragen.

Im Rahmen der Erfindung kann der Begriff hermetisch insbesondere bedeuten, dass die zu prüfende Vorrichtung (beispielsweise das Gehäuse und/oder die elektrische Durchführung oder das Gehäuse mit der elektrischen Durchführung) eine Helium-Leckrate von weniger als 1 × 10⁻⁷ mbar L/sec aufweist. In einer vorteilhaften Ausführung kann die Helium-Leckrate weniger als 1 × 10⁻⁹ mbar L/sec, insbesondere weniger als 1 × 10⁻¹⁰ mbar L/sec betragen.

Die erwähnten Gehäusehalbschalen bestehen zumeist aus einem Metall (z. B. aus Titan Grade 1, Titan Grade 2, Titan Grade 3, 316L, Molybdän Grade 2, o. ä). Dies sorgt für eine sehr gute elektromagnetische Schirmung und schützt die empfindliche Elektronik vor Magnetfeldern und elektromagnetischer Strahlung. Ferner kann die Elektronik mittels Harzen oder anderen Kunststoffen gekapselt werden. Die Aufgabe, elektrische und/oder magnetische Felder von der Platine fernzuhalten oder umgekehrt die Umgebung vor den von dem medizinischen Gerät ausgehenden Feldern zu schützen, muss dann beim Design der Platine und der Auswahl der Komponenten berücksichtigt werden. Bei medizinelektronischen Geräten mit externen Sensoren oder Elektroden werden die Gehäusehalbschalen zusätzlich mit einer elektrischen Durchführung gefügt. Die Durchführung ermöglicht den Austausch von elektrischen Signalen vom Inneren des Gehäuses zur Gehäuseaußenseite durch die hermetisch dicht gefügte Verbindung.

Bei Geräten wie Herzschrittmachern oder implantierbaren Kardiovertern besteht eine solche Durchführung in der Regel aus einem massiven metallischen Durchführungs-Flansch, der beispielsweise aus Titan gefräst ist, und einem oder mehreren in diesen eingesetzte/n Isolierkörper/n, in dem/denen wiederum Anschlussstifte eingebettet sind. Der massive Durchführungs-Flansch hat, relativ zum Gewicht anderer wesentlicher Komponenten des Gerätes, ein recht hohes Gewicht.

Die Funktion und Herstellung solch medizinischer Durchführung ist Gegenstand zahlreicher Patentveröffentlichungen (z. B. EP 2 371 418 A2, EP 1 685 874 A1) und wird daher als bekannt vorausgesetzt und nicht näher beschrieben.

Im Interesse eines hohen Tragekomforts und einer hierdurch begünstigten hohen Nutzerakzeptanz soll ein Gerät der oben genannten Art möglichst leicht sein. Da bezüglich der elektrischen und elektronischen Funktionseinheiten nur ein begrenztes Potential für Gewichtseinsparungen besteht, besteht ein Bedarf an möglichst leichten Gerätegehäusen und Durchführungen.

Weiterhin besteht für die Außenhülle, bestehend aus Gehäusehalbschalen und Flansch der elektrischen Durchführung, der genannten Geräte insgesamt die Anforderung, dass diese bei Magnetresonanz-Untersuchungen des Patienten (MRT, engl. MRI) einerseits die im Gehäuse aufgenommenen Funktionseinheiten möglichst zuverlässig vor dem Einfluss der starken statischen und hochfrequenten Magnetfelder schützen und andererseits bei den Untersuchungen möglichst wenig Störungen zulassen dürfen. Insbesondere sollen sich die Bauteile in starken Magnetfeldern möglichst nicht oder nur sehr geringfügig erwärmen.

Diese wesentlichen Aspekte werden in der MRT-Sicherheitsprüfung von medizinischen Implantaten und Instrumenten nach Norm ASTM F2182 "Standard Test Method for Measurement of Radio Frequency Induced Heating On or Near Passive Implants During Magnetic Resonance Imaging" beschrieben. So ist es für die Zulassung von Medizinprodukten erforderlich, die Erwärmung des Gewebes auf einen zulässigen Wert zu begrenzen. Da das Signal-Rauschverhältnis bei größeren Feldstärken besser wird, setzt die Diagnostik zunehmend auf Ultra-Hochfeld-Magnetresonanztomographie mit mehr als 3T Feldstärke. Um auch Patienten mit Implantaten die Fortschritte der bildgegebenen Untersuchung zu ermöglichen, muss das Implantat so gestaltet sein, dass die Wirbelströme [eddy currents] in der Außenhülle möglichst klein sind bzw. das Gewebe um das Implantat durch die Wärme der Wirbelströme [implant heating in MRI] nicht geschädigt wird.

Die obigen Aufgaben werden durch ein metallisches Außenhüllenteil mit den Merkmalen des Anspruchs 1 besonders gut gelöst. Zweckmäßige Fortbildungen des Erfindungsgedankens sind Gegenstand der abhängigen Ansprüche. Insbesondere wird ein Durchführungs-Flansch mit den Merkmalen des Anspruchs 13 bereitgestellt. Weiterhin wird ein implantierbares medizinelektronisches Gerät mit den Merkmalen des Anspruchs 15 bereitgestellt.

Die Erfindung schließt den Gedanken ein, vorzugsweise in der Außenhüllenwand des Außenhüllenteils der genannten Geräte, konstruktiv Hohlräume vorzusehen, die einerseits zu einer Gewichtseinsparung führen und andererseits das Fließen von Wirbelströmen behindern. Ferner kann der gleiche Effekt durch den Einschluss von nichtleitenden Substanzen (z. B. ein Gas wie Luft oder ein inertes Gas) oder nichtleitenden Partikeln (nicht-metallisch, anorganisch, z. B. Aluminiumoxid, Titanoxid, Siliziumoxid, Glas) erreicht werden. Diese Substanzen oder Partikeln weisen bevorzugt eine geringere Dichte als der Hüllenwerkstoff bzw. als die Hüllenwerkstoffe auf. Diese Substanzen oder Partikeln weisen bevorzugt eine geringere Dichte als der Hüllenwerkstoff auf. Weiterhin gehört zur Erfindung der Gedanke, solche Hohlräume und/oder nichtleitende Einschlüsse zur vorgesehenen Geräteaußenseite und/oder Geräteinnenseite hin mit einer geschlossenen Metallschicht abzuschließen, damit die hermetische Dichtigkeit der Außenhülle gewährleistet bleibt. Mit diesen Gedanken lassen sich sowohl eine Gewichtseinsparung als auch vorteilhafte Eigenschaften des Gerätes bei MRT- und ähnlichen Untersuchungen erzielen, ohne dass sich sonstige Eigenschaften, insbesondere die Langzeit-Zuverlässigkeit, verschlechtern.

Ein innerer Hohlraum und/oder nichtleitender Einschluss in einer Wand ist ein Innenraum, der allerseits von der Wand der Außenhülle umschlossen ist, auch wenn das medizinische Gerät (Herzschrittmacher, Defibrillator, Nervenstimulator) selbst noch nicht gefügt oder endmontiert wurde. So müssen die Hohlräume und/oder nichtleitenden Einschlüsse bereits inhärent in den Konstruktionsbauteilen (Flansch, Gehäusehalbschalen) eingebracht werden. Während der Herstellung wird das Konstruktionsbauteil aus einem Grundwerkstoff (z.B. Titan Grade 1, Titan Grade 2, Titan Grade 4, Titan Grade 5, Nitinol, Ti-6Al-4, Ti-6Al-7Nb, Ti-5Al-2,5Fe, Mo, Nb, Pt) gefertigt und dabei in die Wand des Bauteils Hohlräume mit einer gasförmigen Füllung (z.B. Atmosphäre, Argon, Helium, Wasserstoff) und/oder isolierende Partikel (z.B. Aluminiumoxid, Titanoxid, Siliziumoxid, Wolframcarbit, Aluminiumnitrit, o.ä.) eingebracht.

Die inneren Hohlräume weisen bevorzugt ein Volumen zwischen 5,0 × 10⁻⁵ mm³ und 5,0 x 10⁻² mm³ auf und/oder sind Pore mit einem Durchmesser bevorzugt von 50µm - 500 µm.

In einer Ausführung der Erfindung umfasst zumindest ein Abschnitt des Außenhüllenteils eine Mehrzahl von stofflich, insbesondere einstückig, miteinander verbundenen Schichten, wobei mindestens eine innere Schicht eine Vielzahl von kleinen Hohlräumen oder Partikeln aus einem isolierendem Material aufweist. Die besagte Schicht oder Schichten kann/können grundsätzlich hochgradig porös, also zum Beispiel durch einen Metallschaum gebildet sein. Sie kann/können aber insbesondere auch definiert lokal eingebrachte Hohlräume oder Isolationskörper enthalten. Aus derzeitiger Sicht bevorzugt ist eine Ausführung mit mindestens einer inneren Schicht, die eine reguläre Konfiguration an kleinen Hohlräumen oder an Isolationskörpern aufweist. Eine weitere Ausführung weist eine irrreguläre Verteilung der Hohlräume bzw. Isolationskörper innerhalb der Außenhüllenwand auf.

In einer weiteren bevorzugten Ausführung hat das Außenhüllenwandteil mehrere übereinanderliegende innere Schichten, die verschiedene Anordnungen an kleinen Hohlräumen mit unterschiedlicher Hohlraumgröße und/oder Form und/oder unterschiedlichen Hohlraumabständen haben. Die Hohlräume haben eine Ausdehnung bevorzugt von 10 bis 500 µm. Es kann also beispielsweise eine der Schichten eine große Anzahl kleiner Hohlräume, eine darüber liegende weitere Schicht eine kleinere Anzahl etwas größerer Hohlräume und eine noch weitere Schicht eine noch kleinere Anzahl noch größerer Hohlräume enthalten, oder es sind alternierend Schichten mit einer größeren oder kleineren Anzahl von Hohlräumen bzw. mit kleineren oder größeren Hohlräumen bzw. mit enger oder weiter beanstandeten Hohlräumen. Das gleiche gilt für Mehrschichtstrukturen mit isolierenden Partikeln. Einerseits können so die statischen Anforderungen an die Gerätehülle zuverlässig erfüllt und andererseits kann eine möglichst effiziente Behinderung von Wirbelströmen erreicht werden.

In weiteren Ausführungen der Erfindung werden in der Außenhülle elektrisch isolierende Partikel zum Beispiel aus nichtmetallisch, anorganischen Werkstoffen (z.B. keramische Werkstoffe) vorgesehen. Besonders vorteilhaft sind keramische Partikel mit einer Größe von ca. 1-250 µm, welche in metallische Werkstoffe eingesetzt werden. Gut geeignet sind Partikel aus keramischem Material (z. B. Al₂O₃, SiO₂) welche eine hohe Biokompatibilität, eine geringere Dichte als Titan haben und sich volumenstabil, ohne Phasenumwandlungen beim Herstellprozess (Sinterung, Wärmebehandlung, Hochtemperaturlöten, o.ä) verhalten.

Die Herstellung der Außenhüllenteile ist mit allen additiven Fertigungsverfahren möglich. An Hand des Siebdrucks, wie zum Beispiel in WO 2014/187567 A2 und WO 1993/016865 A1 beschrieben, soll beispielhaft die Herstellung der Bauteile erklärt werden.

Der Herstellungsprozess beginnt mit der Fertigung von verschiedenen Sieben bzw. Schablonen. Die Siebe können mittels feiner Drähte stabilisiert sein, damit feinere Strukturen erreicht werden können. Mittels Fotolithografie und selektiven Ätzen werden die Flächen in dem Sieb frei gelegt, die später zum Auftragen von Material dienen sollen. Für jede geänderte Geometrie in der Aufbaurichtung (senkrecht zur Auflage) wird jeweils ein Sieb erstellt.

Während des Siebdruckprozesses wird das zu druckende Material auf das Sieb aufgetragen und mittels Rakel über das Sieb verteilt. Hierbei wird jeweils eine Schichthöhe in etwa der Siebhöhe gedruckt. Der Prozess wird mehrmals wiederholt bis das Bauteil vollständig aufgebaut wurde. Um beispielsweise die Ausführungsvariante eines Außenhüllenteils aus Titan mit innenliegenden Kavitäten/ Porenstruktur herzustellen, wird bevorzugt für den Druckprozess eine Paste (z.B. Titan, Niob) hergestellt. Nacheinander wird durch eine Mehrzahl geeignet strukturierter Siebe auf eine geeignete Druckunterlage bzw. auf die jeweils vorangehend erzeugte Schicht gerakelt bzw. gedruckt. Jede Schicht wird vor der neuen Lage, abhängig vom verwendeten Bindersystem, geeignet getrocknet (mittels IR- oder UV-Trockner). Das Titanpulver in der Druckpaste kann beispielhaft eine mittlere Teilchengröße im Bereich zwischen 10 und 30 µm, insbesondere zwischen 10 und 20 µm, haben, und es kann ein Sieb mit einer Maschenöffnung von beispielsweise im Bereich zwischen 50 und 100 µm und mit einem Drahtdurchmesser im Bereich zwischen 15 und 40 µm benutzt werden. Zur Erreichung der gewünschten, inneren Kavitäten ist bevorzugt so vorzugehen, dass mehrere Einzellagen mit gezielten Freiräumen entstehen, die durch verschlossene Siebbereiche erzeugt und anschließend mit einem an diesen Stellen wieder geöffnetem Sieb überdruckt werden. Überraschenderweise hat sich gezeigt, dass wenn man hierzu ein Pastensystem mit scherverdünnenden Eigenschaften verwendet, dass die Paste die entstandenen Hohlräume nicht ausfüllt, sondern überspannt.

Besonders bevorzugt kommt für das Ti-Grundgerüst ein Ti-Pulver zum Einsatz mit möglichst feiner Körnung (z. B. 10-45 µm) und für die Überdeckung der (vor der Deckschichtaufbringung freigemachten) Hohlräume Pulver mit grober Körnung (z. B. 40-150 µm). Alternative zur Überdeckung der Hohlräume mit groben Pulvern können die Kavitäten vor ihrer Überdeckung auch mit den oben beschriebenen keramischen Pulvern gefüllt werden, was eine größere Dimensionierung der Kavitäten auch für die Verfahren EDM und LBM zuließe.

Alternativ zu Hohlräumen können auch Lagen mit erhöhtem Isolatorenanteil (z.B. Al₂O₃, SiO₂, WC) gemischt werden, indem ein Pulver bzw. Paste hergestellt wird, welches aus einem metallischen Pulver und einem Isolator besteht.

Um eine für die weitere Verarbeitung und/ oder Applikation notwendige mechanische Festigkeit zu erreichen, wird die Schichtstruktur nach Fertigstellung unter Schutzgasatmosphäre entbindert und im Hochvakuum bei Temperaturen > 1000 °C gesintert. Gerade auch im Hinblick auf den folgenden Fügeprozess der Außenhüllenteile, beispielsweise mittels Laserschweißens ist über die gesamte Prozesskette auf einen möglichst geringen Sauerstoff-Pick-Up zu achten. Anderweitig kommt es zu einer Versprödung der Schmelzzone und zu einer unerwünschten Degradation der mechanischen Festigkeit über die Nutzungsdauer des Implantats.

In weiteren Ausführungen der Erfindung weist mindestens eine Schicht ein anderes Material als mindestens eine der übrigen Schichten auf. Insbesondere weist hierbei mindestens eine innere Schicht des Schichtverbundes ein anderes Material als die beiden das Außenhüllenteil nach außen begrenzenden Schichten auf. Beispielsweise kann das Außenmaterial (z. B. Titan Grade 1, 2, 4 oder 5, Edelstahl 316L) nach den Anforderungen der Schweißbarkeit gewählt werden, während das Material der innen liegenden Schichten so gewählt werden kann, dass die Gewichtsersparnis möglichst groß und/oder die Unterdrückung von Wirbelströmen möglichst effizient ist, z. B. Aluminium, Kupfer, Titan Grade 2 und Aluminiumoxid. Es ist aber auch möglich, die Schichten im Wesentlichen aus einem einzigen Material bzw. einem Basis-Material mit vergleichsweise geringfügigen Beimischungen zu bilden.

In technologisch einfacher Weise lässt sich das vorgeschlagene Außenhüllenteil auch realisieren als Sinter-Teil, bei dem die Schichten miteinander versintert sind. Die Herstellung derartiger Teile bildet ein eigenes technisches Gebiet, die Pulvermetallurgie, so dass dem Fachmann für die Gehäuseherstellung von medizinelektronischen Geräten umfangreiches Wissen und ausgereifte Technologien bereitstehen, aus denen er im Rahmen fachmännischen Handelns auswählen kann. Eine genauere Beschreibung der Herstellung dieser Ausführungsform ist daher hier verzichtbar.

Zur technologisch einfachen Realisierung der primären Bauteilstruktur ist das Außenhüllenteil insbesondere als thermisch verfestigtes 3D-Druckteil ausgebildet, das mehrere jeweils durch Drucken einer Metallpulverzusammensetzung gebildete Schichten aufweist. Bei dem eingesetzten Druckverfahren kann es sich speziell neben dem bereits beschriebenen Siebdruckverfahren auch um ein originär generatives Druckverfahren nach Art des Jet-Printing handeln.

Dabei können Druckpasten mit verschiedenen Reinheitsgraden, respektive Primärkornstruktur eingesetzt werden. Weiterhin können den Pasten gezielt elektrisch isolierende Bestandteile zugesetzt werden, welche die Leitfähigkeit des Werkstückes lokal verschlechtern.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische, teilweise geschnittene Darstellung eines implantierbaren medizinelektronischen Geräts,
- Fig. 2A, 2B und 2C: eine schematische perspektivische Darstellung bzw. eine Schnittansicht einer Ausführungsform des erfindungsgemäßen Außenhüllenteils bzw. eine Folge von Schnitten in verschiedenen Ebenen dieses Teils, und
- Fig. 3: einen Schnitt durch ein Schrittmachergehäuse mit zugeführten Partikeln im mittleren Bereich der Außenhülle

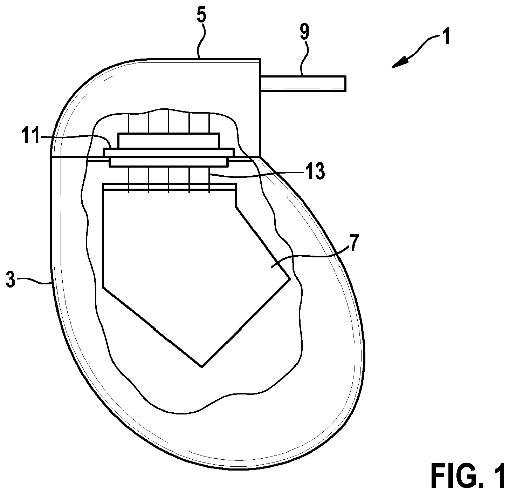

Fig. 1 zeigt schematisch einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11, die in einem Durchführungs-Flansch 12 gehaltert ist, umfasst eine Mehrzahl von Anschlusselementen 13 zum externen Anschluss der Leiterplatte 7.

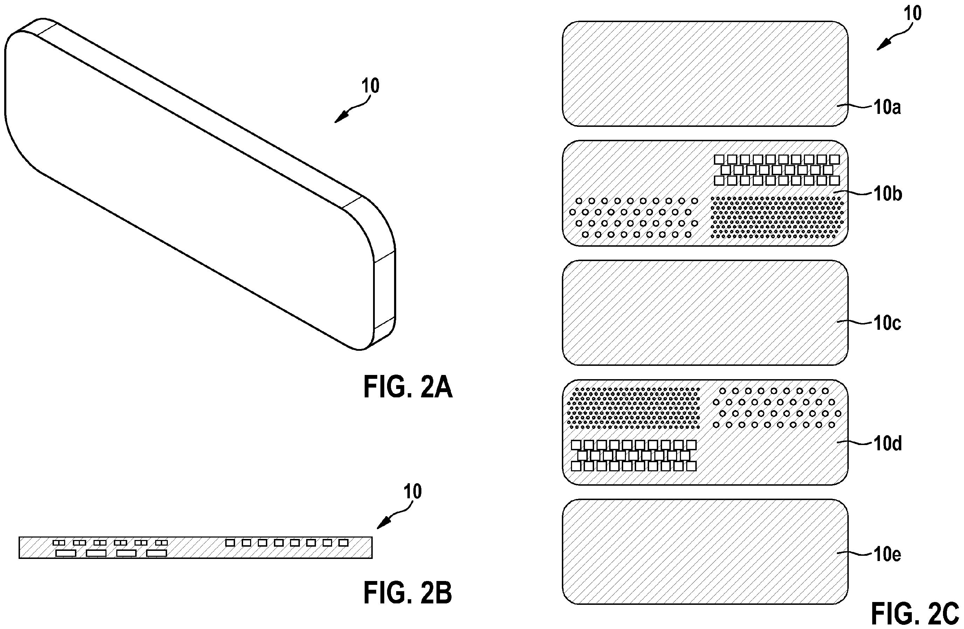

Die Figuren 2A, 2B und 2C zeigen schematisch eine Ausführung eines hier mit Ziffer 10 bezeichneten Außenhüllenteils, welches beispielsweise einen Abschnitt des Gehäuses 3 oder ein Halbfabrikat zur Herstellung eines Durchführungs-Flansches der Durchführung 11 des in Fig. 1 schematisch gezeigten und oben kurz beschriebenen Herzschrittmachers 1 bilden kann.

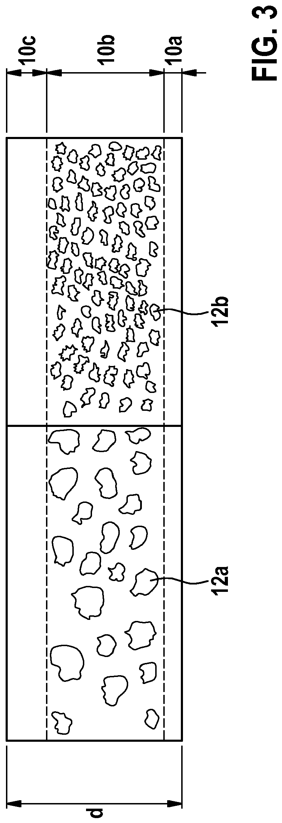

Figur 3 zeigt schematisch einen Schnitt den Querschnitt durch ein Außenhüllteil eines medizinischen Implantats, z. B. eines Schrittmachers. Die oberen Bereiche 10a und die unteren Bereiche 10e sind aus einer geschlossenen Metallschicht, z. B. aus Titan Grade 2 gebildet.

Die mittleren Bereiche 10b besteht aus einem Metall, z. B. Titan Grade 3, mit eingebetteten isolierenden Keramikpartikeln 12a bzw. 12b unterschiedlicher Größe, z. B. aus Al2O3.

Das Außenhüllenteil 10 hat, wie schon in Fig. 2B deutlich wird, sich aber genauer an den Längsschnittdarstellungen einzelner Schichten 10a bis 10e in Fig. 2C ablesen lässt, einen Schichtaufbau aus beispielhaft fünf miteinander stofflich verbundenen Schichten. Hiervon sind die oberste Schicht 10a und die unterste Schicht 10e sowie eine mittlere Schicht 10c geschlossene Metallschichten, während die dazwischenliegenden Schichten 10b und 10d jeweils drei Gruppen von regulär angeordneten Hohlräumen aufweisen. Die unterschiedlichen Hohlräume unterhalb dieser Schichten sind lediglich als Illustrationen zu verstehen, und auch die unterschiedliche Anordnung der Hohlräume in den beiden Schichten 10b und 10d ist lediglich beispielhaft zu verstehen. In der Praxis werden eher Ausführungen Anwendung finden, in denen Hohlräume mit einer bestimmten Größe, Form und bestimmtem Abstand über die gesamte Schicht verteilt sind, während in einer anderen Schicht Hohlräume einer anderen Größe, Form und/oder mit anderen Abständen vorgesehen sein werden, um mit dem Schichtverbund insgesamt bestimmte Stabilitäts- und elektrische Anforderungen zu erfüllen. Die Schichtabfolgen 10b, 10c, 10d können sich mehrmals in unterschiedlicher Reihenfolge wiederholen bevor die Struktur mit 10a und 10e abgeschlossen wird. Wie in 10b zu erkennen sind insbesondere Waben-, Kreis-, Rechteck, Kugel-, Zylinderstrukturen von Vorteil.

Die gezeigten Strukturen der Schichten 10b und 10d können beispielsweise mittels einer entsprechend gestalteten (etwa in einem Fotolitografieverfahren erzeugten) Siebdruckschablone in einem Siebdruckverfahren oder auch durch entsprechende Ansteuerung eines Jet-Drucksystems erzeugt werden. Die typische Strukturgröße bei den beschriebenen Prozessen beträgt 1-30 µm.

Im Übrigen ist die Ausführung der Erfindung auch in einer Vielzahl von Abwandlungen der hier gezeigten Beispiele und weiter oben hervorgehobenen Aspekte der Erfindung möglich.

Zum Beispiel ist es vorstellbar, durch den gezielten Einsatz von feineren Ti-Pulvern und feineren Sieben, noch feinere Kavitätenstrukturen zu gestalten.

Technologisch alternativ zum Siebdruck kann zur Herstellung der beschriebenen Strukturen im Außenhüllenteil auch auf weitere additive Formgebungsverfahren zurückgegriffen werden, wie z.B. Spritzguss (MIM, PIM). Hierbei wird zuerst mit einem Granulat in einer hohen Materialreinheit vorgespritzt. Anschließend wird das Bauteil mittels eines zweiten Granulats mit einem Anteil von Triebmitteln und/oder keramischen Anteil zu Ende gespritzt. Während des Sinterns kann das Triebmittel die Hohlräume erzeugen und die Keramischen Einschlüsse werden fest in das Gefüge eingebaut.

Auch weitere 3D-Druckverfahren (z.B. Ink-Jet, Filamentdruck) oder auch SLM oder EBM. Allerdings ist gerade bei der Verwendung von Elektronen- oder Laserstrahlverfahren auf die Verwendung von geeigneten Pulvern zu achten, die das Titangrundgerüst ausbilden. Bei der Wahl des LBM oder EBM kann es allerdings zu einer Einschränkung der Freiheitsgrade bei der Ausgestaltung der Kavitäten Größe und Dicke der die Hohlräume überdeckenden Schicht kommen.

Schließlich werden im Folgenden weitere Aspekte der vorliegenden Erfindung und Ausführungsformen dieser Aspekte als Gegenstände formuliert, wobei diese Gegenstände auch als Ansprüche der vorliegenden Erfindung formuliert werden können. Insbesondere beziehen sich die Bezugszeichen in Klammern auf die Figuren.
Gegenstand 1: metallisches Außenhüllenteil (10) eines implantierbaren medizinelektronischen Gerätes (1), das mindestens einen inneren Hohlraum und/oder nichtleitende Einschluss oder Abschnitt (10b, 10d) mit einer Vielzahl kleiner Hohlräume und/oder nichtleitende Einschlüsse (12a, 12b) aufweist, der mindestens zur Geräteaußenseite hin mit einer geschlossenen Metallschicht (10a, 10e) hermetisch dicht abgeschlossen ist.
Gegenstand 2: Außenhüllenteil nach Gegenstand 1, das eine Mehrzahl von stofflich einstückig miteinander verbundenen Schichten (10a - 10e) umfasst, wobei mindestens eine innere Schicht (10b, 10d) eine Vielzahl von kleinen Hohlräumen und/oder nichtleitende Einschlüsse (12a, 12b) aufweist.
Gegenstand 3: Außenhüllenteil nach Gegenstand 2, wobei die oder mindestens eine innere Schicht (10b, 10d) aus aufgeschäumtem metallischem Werkstoff gebildet ist.
Gegenstand 4: Außenhüllenteil nach Gegenstand 2, wobei die oder mindestens eine innere Schicht (10b, 10d) eine reguläre Konfiguration an kleinen Hohlräumen aufweist.
Gegenstand 5: Außenhüllenteil nach einem der Gegenstände 2 bis 4, mit mehreren übereinanderliegenden inneren Schichten (10b - 10d), die verschiedene Anordnungen an kleinen Hohlräumen mit unterschiedlicher Größe und/oder Form und/oder unterschiedlichen und/oder nichtleitenden Einschlüssen haben.
Gegenstand 6: Außenhüllenteil nach einem der Gegenstände 2 bis 5, ausgebildet als Sinter-Teil (10), bei dem die Schichten miteinander versintert sind.
Gegenstand 7: Außenhüllenteil nach einem der Gegenstände 2 bis 6, wobei mindestens eine Schicht ein anderes Material als mindestens eine der übrigen Schichten aufweist.
Gegenstand 8: Außenhüllenteil nach Gegenstand 7, wobei mindestens eine innere Schicht (10b - 10d) ein anderes Material als die beiden das Außenhüllenteil nach außen begrenzenden Schichten (10a, 10e) aufweist.
Gegenstand 9: Außenhüllenteil nach Gegenstand 7 oder 8, wobei das andere Material der mindestens einen Schicht sich in Dichte und/oder Leitfähigkeit von dem Material der übrigen Schichten unterscheidet.
Gegenstand 10 Außenhüllenteil nach einem der vorangehenden Gegenständen, ausgebildet als thermisch verfestigtes 3D-Druckteil (10), das mehrere jeweils durch Drucken einer Metallpulverzusammensetzung gebildete Schichten (10a - 10e) aufweist.
Gegenstand 11: Außenhüllenteil nach einem der vorangehenden Gegenständen, ausgebildet als Durchführungs-Flansch (10) mit mindestens einer Durchgangsöffnung für ein Anschlusselement oder einen Isolierkörper zur isolierten Aufnahme von Anschlusselementen.
Gegenstand 12: Außenhüllenteil nach einem der Gegenstände 1 bis 10, ausgebildet als Gehäusehalbschale oder Teil einer Gehäusehalbschale (3) eines implantierbaren medizinelektronischen Gerätes (1).
Gegenstand 13: Medizinelektronisches Gerät (1), insbesondere Herzschrittmacher, Kardioverter, Cochlearimplantat oder Neurostimulator mit einem metallischen Außenhüllenteil (10) nach einem der vorangehenden Gegenstände.

## Patentansprüche

1. Metallisches Außenhüllenteil (10) für ein implantierbares medizinelektronisches Gerät (1), wobei das metallische Außenhüllenteil (10) innerhalb der Außenhüllenwand mindestens einen inneren Hohlraum und/oder nichtleitenden Einschluss aufweist, wobei der mindestens eine Hohlraum und/oder nichtleitende Einschluss mindestens zur vorgesehenen Geräteaußenseite hin mit einer geschlossenen Metallschicht (10a, 10e) hermetisch dicht abgeschlossen ist.

2. Metallisches Außenhüllenteil (10) nach Anspruch 1, wobei der mindestens eine Hohlraum und/oder nichtleitende Einschluss mindestens zur vorgesehenen Geräteinnenseite hin mit einer geschlossenen Metallschicht (10a, 10e) hermetisch dicht abgeschlossen ist.

3. Metallisches Außenhüllenteil (10) nach einem der vorangehenden Ansprüche, wobei das metallische Außenhüllenteil (10) innerhalb der Außenhüllenwand mindestens einen Abschnitt (10b, 10d) mit einer Vielzahl kleiner Hohlräume und/oder nichtleitender Einschlüsse (12a, 12b) innerhalb der Außenhüllenwand aufweist.

4. Außenhüllenteil (10) nach einem der vorangehenden Ansprüche, wobei zumindest ein Abschnitt der Außenhüllenwand eine Mehrzahl von stofflich einstückig miteinander verbundenen Schichten (10a - 10e) umfasst, wobei mindestens eine innere Schicht (10b, 10d) eine Vielzahl von kleinen Hohlräumen und/oder nichtleitenden Einschlüssen (12a, 12b) aufweist.

5. Außenhüllenteil (10) nach Anspruch 4, wobei die oder mindestens eine innere Schicht (10b, 10d) aus aufgeschäumtem metallischem Werkstoff gebildet ist.

6. Außenhüllenteil (10) nach Anspruch 4, wobei die oder mindestens eine innere Schicht (10b, 10d) eine reguläre Konfiguration an kleinen Hohlräumen und/oder Einschlüssen aufweist.

7. Außenhüllenteil (10) nach einem der Ansprüche 4 bis 6, wobei mehrere übereinanderliegende innere Schichten (10b - 10d) verschiedene Anordnungen an kleinen Hohlräumen und/oder Einschlüssen mit unterschiedlicher Größe und/oder Form haben.

8. Außenhüllenteil (10) nach einem der Ansprüche 4 bis 7, wobei der zumindest eine Abschnitt der Außenhüllenwand als Sinter-Teil (10), bei dem die Schichten miteinander versintert sind, ausgebildet ist.

9. Außenhüllenteil (10) nach einem der Ansprüche 4 bis 8, wobei mindestens eine Schicht ein anderes Material als mindestens eine der übrigen Schichten aufweist.

10. Außenhüllenteil (10) nach Anspruch 9, wobei mindestens eine innere Schicht (10b - 10d) ein anderes Material als die beiden das Außenhüllenteil nach außen begrenzenden Schichten (10a, 10e) aufweist.

11. Außenhüllenteil (10) nach Anspruch 9 oder 10, wobei das andere Material der mindestens einen Schicht sich in Dichte und/oder Leitfähigkeit von dem Material der übrigen Schichten unterscheidet.

12. Außenhüllenteil (10) nach einem der Ansprüche 4 bis 11, wobei der zumindest eine Abschnitt der Außenhüllenwand als thermisch verfestigtes 3D-Druckteil (10), das mehrere jeweils durch Drucken einer Metallpulverzusammensetzung gebildete Schichten (10a - 10e) aufweist, ausgebildet ist.

13. Außenhüllenteil (10) nach einem der vorangehenden Ansprüche, ausgebildet als Durchführungs-Flansch (10) mit mindestens einer Durchgangsöffnung für ein Anschlusselement oder einen Isolierkörper zur isolierten Aufnahme von Anschlusselementen.

14. Außenhüllenteil (10) nach einem der Ansprüche 1 bis 12, ausgebildet als Gehäusehalbschale oder Teil einer Gehäusehalbschale (3) eines implantierbaren medizinelektronischen Gerätes (1).

15. Medizinelektronisches Gerät (1), insbesondere Herzschrittmacher, Kardioverter, Cochlearimplantat oder Neurostimulator mit einem metallischen Außenhüllenteil (10) nach einem der vorangehenden Ansprüche.
